# EUROPÄISCHE PATENTSCHRIFT

(11) **EP 0 552 496 B1**
(45) Veröffentlichungstag und Bekanntmachung des Hinweises auf die Patenterteilung: **16.08.1995**
(21) Anmeldenummer: 92122022.4
(22) Anmeldetag: 28.12.1992
(51) Int. Cl.: C02F 3/28

(54) **Modul für einen Reaktor zur anaeroben Reinigung von Abwasser**
Module for a reactor for anaerobic waste water purification
Module pour un réacteur pour la purification anaérobie d'eau usée

(30) Priorität: 24.01.1992 DE 4201864
(43) Veröffentlichungstag der Anmeldung: 28.07.1993
(73) Patentinhaber: PASSAVANT-WERKE AG, 65322 Aarbergen 7 (DE)
(72) Erfinder: Zumbrägel, Michael, Dipl.-Ing., W-6209 Aarbergen 2 (DE); Bach, Volker, W-6209 Aarbergen 1 (DE)

(56) Entgegenhaltungen:
- EP-A- 0 193 999
- EP-A- 0 244 029
- FR-A- 2 266 739
- NL-A- 8 101 821
- H20 Bd. 24, Nr. 13, Juli 1991, RIJSWIJK, NL Seiten 356 - 361 J. J. BOGTE ET AL. *Seite 357, rechte Spalte, Absatz 2, Zeilen 1-2*

## Beschreibung

Die Erfindung betrifft ein Modul für einen Reaktor zur anaeroben Reinigung von Abwasser.

Bei der anaeroben Abwasserbehandlung werden die organischen Abwasserinhaltsstoffe zu den Endprodukten Methan (CH₄) und Kohlendioxid (CO₂) abgebaut. Ein Teil wird in die Zellsubstanz der Mikroorganismen eingebaut und muß später als Überschußschlamm abgezogen werden.

Das in dem Reaktor aufsteigende, methanhaltige Biogas muß aufgefangen und abgeleitet werden. Wegen des hohen Brennwerts wird es zur Strom-, Kraft- oder Wärmegewinnung benutzt.

Das Auffangsystem besteht aus mehreren Lagen übereinander angeordneter Hauben, die vollständig geflutet sind. An den Außenflächen der Hauben setzen sich eventuell mit hochgerissene Schlammpartikel ab und sinken in den Reaktor zurück. Infolge der Abtrennung der Phasen Biogas und Wasser von der Phase "Bioschlamm" nennt man dieses Kernstück des Reaktors "Drei-Phasen Trennsystem".

Dieses Trennsystem wird in Modulen mit rechteckiger Außenform gefertigt. Die Module bestehen aus verschiedenen Werkstoffen, wobei bei der Wahl der Werkstoffe auf die in diesem Bereich herrschende korrosive Athmosphäre und die Abdichtverhältnisse Rücksicht genommen wurde (Prospekt "WSI 92/89 Anaerobe Abwasserreinigung BIOPAQ" der Passavant-Werke AG). Die Verwendung verschiedener Werkstoffe mit verschiedenen Temperaturgradienten und der Einbau von Holz als tragende Struktur führen dazu, daß Undichtigkeiten und Geruchsemissionen entstehen. Die Verbindungselemente der Bauteile sind nicht korrosionsfest.

Biogas, Abluft und gereinigtes Abwasser werden unter der Unterkante der Module unterhalb des Wasserspiegels abgeführt. Die Folge davon ist, daß bei notwendigen Reperaturarbeiten der Reaktor zumindest teilweise abgelassen werden muß. Dies führt zu unnötig langen Stillstandszeiten.

Die Aufgabe, hier wirksame Abhilfe zu schaffen, wird gemäß der Erfindung dadurch gelöst, daß alle Wandungen, Abdeckungen und Einbauteile des Moduls aus ggf. mit Stahlprofilen verstärktem Kunststoff bestehen und miteinander verschweißt sind, und daß alle Ableitungen an einer Schmalseite des Moduls münden und mit dort längs mehrerer Module verlegten, abschnittsweise verschließbaren Entsorgungskanälen gasdicht verbunden sind. Diese neue Konstruktion vermeidet jegliche Verbindungselemente. Alle Schweißstellen der Stahlprofilverstärkungen sind korrosionssicher von Kunststoff überdeckt. Alle Flächen, die mit den korrosiven Medien in Berührung kommen, sind aus einem korrosionsfesten Material gefertigt. Die Abdeckung des Moduls besteht aus dem gleichen Material, so daß Undichtigkeiten infolge unterschiedlicher Temperaturdehnung nicht auftreten können. Die Abführung von Biogas, Abluft und gereinigtem Abwasser erfolgt jetzt durch einen neben den Modulen verlaufenden gesonderten Entsorgungsschacht, der vom Innenraum des Moduls völlig getrennt ist. Infolgedessen braucht bei Reperaturarbeiten im Schacht der Reaktor nicht abgelassen zu werden. Alle im Schacht verlegten Rohrleitungen sind leicht zugänglich und ermöglichen infolge der Unterteilung in absperrbare Abschnitte einfache Reperatur- und Wartungsarbeiten.

Vorteilhafterweise erhält jedes Modul ein über seine ganze Tiefe durchlaufendes vorspringendes Abteil für die Sammlung des Biogases. Über diesem Abteil liegt der Entsorgungsschacht, in dem die Rohrleitungen im wesentlichen nebeneinander und damit leicht zugänglich verlegt sind.

Weitere bevorzugte Konstruktionsmerkmale sind der nachfolgenden Beschreibung eines in den Zeichnungen dargestellten Ausführungsbeispiels zu entnehmen. Es zeigen im einzelnen:
- Fig. 1: die Draufsicht auf einen mit vier Modulen ausgestatteten Abschnitt eines Anaerob-Reaktors,
- Fig. 2: einen Vertikallängsschnitt durch ein Modul nach Fig.1,
- Fig. 3: eine schräge Draufsicht auf die geöffnete Oberseite eines perspektivisch dargestellten Moduls nach Fig. 1,
- Fig. 4: einer Vertikalquerschnitt durch das Modul nach Fig. 1,
- Fig. 5: einen Querschnitt durch den von zwei Modulen begrenzten Entsorgungsschacht.

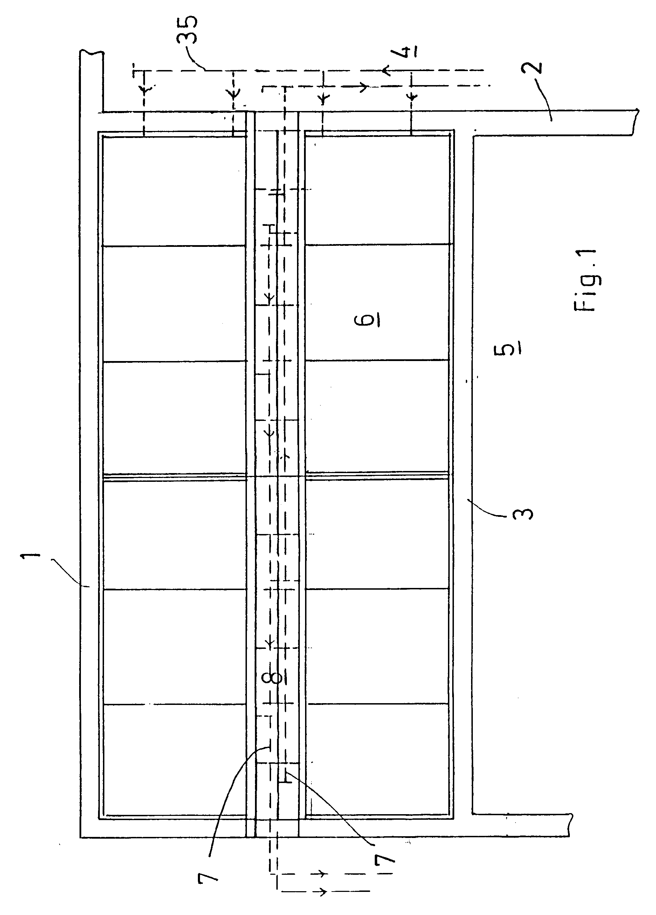

In der Draufsicht nach Fig. 1 ist die Außenwand des Reaktors mit 1 bezeichnet. Zwischenwände 2 und 3 trennen zum zentralen Entsorgungsabteil 4 und zum benachbarten Abschnitt 5 ab. Jedes Modul ist durch drei Deckel 6 abgedeckt. Es ist auch schon zu erkennen, daß die Module an ihren Längsseiten Vorsprünge 7 haben, die sich gegenüberliegend zusammenstoßen und darüber den Entsorgungsschacht 8 bilden.

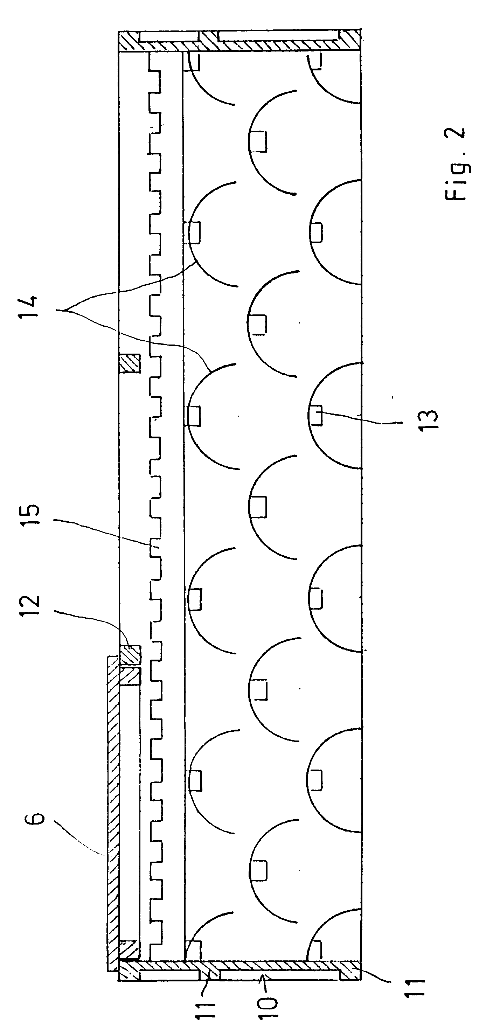

Aus Fig. 2 ist der Aufbau eines Moduls zu erkennen. Die Seitenwände 10 bestehen aus Verstärkungsprofilen 11, die vollständig von dem als Wandmaterial dienenden Kunststoff überdeckt sind. Oben sind zwei Traversen 12 vorgesehen, die den Deckeln 6 als dichtende Auflage dienen. In der sichtbaren Längswand ist eine Vielzahl von durch Taschen 13 abgedeckten Austrittsöffnungen für das Biogas vorgesehen. Die Austrittsöffnungen liegen im Scheitel von Gasauffangauben 14, die hier im Querschnitt halbkreisförmig sind. Die Auffanghauben sind in drei Lagen überlappend angeordnet, so daß Biogas nicht in die Abluftatmosphäre gelangen kann.

Über der obersten Lage der Auffanghauben verläuft die Klarwasser-Überlaufrinne 15, die hier eine gezahnte Einlaufkante hat.

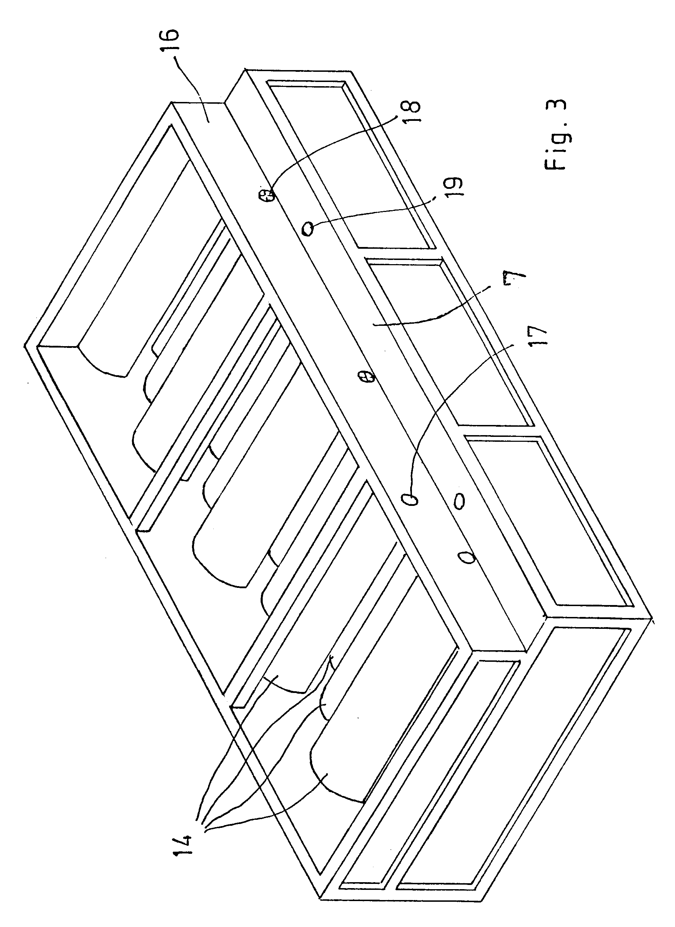

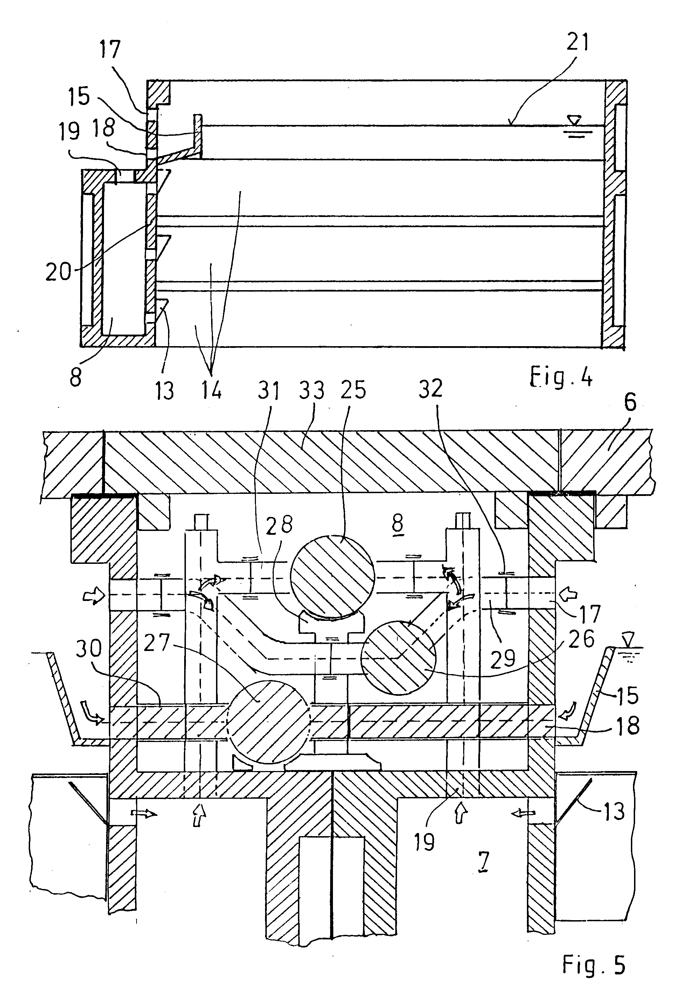

Aus Fig. 3 ist zu ersehen, daß in der über dem vorspringenden Abschnitt 7 liegenden Wandpartie 16 Abzugsöffnungen für Abluft 17 und Klarwasser 18 liegen. Das in dem vorspringenden Abschnitt 7 gesammelte Biogas wird nach oben durch Öffnungen 19 abgezogen. Dies ist auch aus Fig. 4 zu erkennen. Der vorspringende Abschnitt 7 ist gegen den Innenraum des Moduls durch eine Zwischenwand 20 abgeteilt. Die Klarwasser-Überlaufrinne 15 ist für den Wasserspiegel 21 im Reaktor verantwortlich.

Fig. 5 zeigt zwei mit ihren vorspringenden Abschnitten 7 dichtend aneinanderstoßende Module. Die beiden vorspringenden Abschnitte 7 lassen darüber einen Entsorgungskanal 8 frei, in dem die drei Rohrleitungen zum Ableiten von Biogas 25, Abluft 26 und Klarwasser 27 verlegt sind. Zwei der Rohrleitungen liegen auf Konsolen 28 auf, so daß sie abschnittsweise ausgebaut werden können, um an den darunterliegenden Leitungsstrang zu kommen. Die Rohrverbindungen 29, 30, 31 sind mittels muffenloser Spannschellen 32 angeschlossen, wobei die um das Abluft-Sammelrohr 26 herumgeführte Biogas-Rohrverbindung als T-Rohr mit einem oberen Anschluß für Mengen- oder Schadstoffsonden ausgebildet ist. Für den Entsorgungskanal 8 gibt es besondere Abdeckungen 33.

Der Entsorgungskanal 8 mündet in einen Hauptkanal 34 (Fig. 1), der auch die Zuleitungen 35 für das zu behandelnde Abwasser enthält.

## Patentansprüche

1. Modul für einen UASB-Reaktor zur anaeroben Reinigung von Abwasser, enthaltend eine den Wasserstand im Modul festlegende obere Überlaufschwelle für das gereinigte Abwasser, mehrere über den ganzen Modulquerschnitt gestaffelt angeordnete getauchte Auffanghauben für das Biogas mit Ableitung in eine Sammelleitung, und eine obere Abzugsleitung für die über dem Wasserstand anfallende Abluft, **dadurch gekennzeichnet**, daß alle Wandungen (10), Abdeckungen (6, 33) und Einbauteile (13, 14, 15) des Moduls (10) aus ggf. mit Stahlprofilen (11) verstärktem Kunststoff bestehen und miteinander verschweißt sind, und daß alle Ableitungen (17, 18, 19) an einer Schmalseite des Moduls münden und mit außerhalb des Moduls verlegten, abschnittweise verschließbaren Entsorgungskanälen (25, 26, 27) gasdicht verbunden sind.

2. Modul nach Anspruch 1, **dadurch gekennzeichnet**, daß für die Sammlung des Biogases an dieser Schmalseite ein über die ganze Tiefe des Moduls verlaufendes vorspringendes Abteil (7) vorgesehen ist, über dem der Entsorgungskanal (8) verlaüft.

3. Modul nach Anspruch 1 oder 2, **dadurch gekennzeichnet**, daß die Sammelleitungen (25, 26, 27) im Entsorgungskanal (8) durchlaufend ausgebildet und mit lösbaren und absperrbaren Anschlußstutzen (29, 30, 31) versehen sind.

4. Modul nach Anspruch 3, **dadurch gekennzeichnet**, daß die lösbaren Verbindungen zwischen den Anschlußstutzen (29, 30,31) und den Sammelleitungen (25, 26. 27) als muffenlose Rohrschellen (32) ausgebildet sind.

5. Modul nach Anspruch 3 oder 4, **dadurch gekennzeichnet**, daß die Sammelleitungen (25, 26, 37) mittels mehrerer, jeweils die Tiefe eines Moduls übertreffender Konsolen (28) auf den vorspringenden Abschnitten (7) abgestützt sind.

6. Modul nach Anspruch 1 bis 5, **dadurch gekennzeichnet**, daß die Auffanghauben (14) als längshalbierte Kunststoffrohre ausgebildet sind.

7. Modul nach Anspruch 6, **dadurch gekennzeichnet**, daß die Auffanghauben (14) durch Kleben/Schweißen mit den Stirnwänden der Module (10) verbunden sind.

## Claims

1. Module for an UASB-reactor for anaerobic treatment of waste water, comprising an upper overflow weir for clarified water which determines the water level in the module, several immersed collecting hoods for biogas, staggered over the entire module cross section including an exhaust pipe leading into a collecting pipe and an upper discharge pipe for the exhaust air forming on top of the water surface, **characterized in that** all walls (10), covers (6, 33) and internal components (13, 14, 15) of the module (10) are made, if necessary, of steel profile reeinforced with plastic and are welded together, and that all discharge pipes (17, 18, 19) end at one small side wall of the module and are connected gastight to discharge channels (25, 26, 27), which are lockable in sections, provided outside of the module.

2. Module according to claim 1, **characterized in that** a protruding section (7), reaching down the total depth of the module and supporting the discharge channel (8) on its top, is provided at that small side wall for collecting the biogas.

3. Module according to claim 1 or 2, **characterized in that** the collecting pipes (25, 26, 27) continue into the discharge channel (8) and are provided with detachable and lockable connecting sockets (29, 30, 31).

4. Module according to claim 3, **characterized in that** the detachable connections between the flanges (29, 30, 31) and the collecting pipes (25, 26, 27) are clamps (32) without pipe sockets.

5. Module according to claim 3 or 4, **characterized in that** the collecting pipes (25, 26, 27) are supported by several heavy consoles (28) each reaching deeper down than the depth of one module, against the protruding sections (7).

6. Module according to claims 1 through 5, **characterized in that** the collecting hoods (14) are shaped as plastic pipes cut lengthwise in two.

7. Module according to claim 6, **characterized in that** the collecting hoods (14) are glued/welded to the front walls of the module (10).

## Revendications

1. Module pour un réacteur-UASB pour le traitement anaérobie des eaux résiduaires, comprenant un bord-déversoir supérieur pour l'eau clarifiée déterminant le niveau d'eau dans le module, plusieurs capots collecteurs immergés pour le biogas arrangés en zig-zag sur la section transversale entière de module incluyant un tuyau de sortie de gas dirigé dans le tuyau collecteur, et un tuyau de sortie de gaz supérieur pour l'air rejeté en-dessus de la surface d'eau, **caractérisé en ce que** tous les parois (10), couvertures (6, 33) et parties intégrantes (13, 14, 15) du module (10) sont faits, s'il en est besoin, d'acier profilé, renforcé de matières plastique et soudés ensemble, et que tous les tuyaux de sortie de gaz (17, 18, 19) débouchent à un petit côté du module où ils sont reliés de façon imperméable aux gaz aux conduites de décharge (25, 26, 27) pourvues en dehors du module et qui peuvent être obturées par sections.

2. Module selon revendication 1, **caractérisé en ce** que pour la collection du biogas ce petit côté a été pourvu d'une section (7) saillante s'étendant tout le long de la profondeur du module et sur laquelle passe la conduite de décharge (8).

3. Module selon les revendiations 1 ou 2, **caractérisé en ce que** les tuyaux collecteurs (25, 26, 27) continuent dans la conduite de décharge (8) et sont munis de raccords (29. 30, 31) amovibles et verrouillables.

4. Module selon revendication 3, **caractérisé en ce que** les unions détachables entre les raccords (29, 30, 31) et les tuyaux collecteurs (25, 26, 27) sont des colliers d'attache (32) sans manchons.

5. Module selon les revendications 3 ou 4, **caractérisé en ce que** les tuyaux collecteurs (25, 26, 27) sont appuyés par plusieurs consoles (28) chacune s'étendant plus profondement qu'un module, sur les sections saillantes (7).

6. Module selon les revendications 1 à 5, **caractérisé en ce que** les capots collecteurs (14) sont formés comme des tuyaux en matière plastique coupés en deux en long.

7. Module selon revendication 6, **caractérisé en ce que** les capots collecteurs (14) sont collés/soudés aux parois d'extrémité des modules (19).
